# EUROPÄISCHE PATENTSCHRIFT

(11) **EP 1 301 767 B1**
(45) Veröffentlichungstag und Bekanntmachung des Hinweises auf die Patenterteilung: **06.10.2004**
(21) Anmeldenummer: 01956539.9
(22) Anmeldetag: 17.07.2001
(51) Int. Cl.: G01N 1/00

(54) **VERFAHREN ZUR SELEKTIVEN BESTIMMUNG VON BLOCKIERENDEN AUTOANTIKÖRPERN GEGEN DEN TSH-REZEPTOR**
METHOD FOR THE SELECTIVE DETERMINATION OF BLOCKING AUTO-ANTIBODIES TO THE TSH RECEPTOR
PROCEDE DE DETERMINATION SELECTIVE D'ANTICORPS BLOQUANTS CONTRE LE RECEPTEUR DE LA THYREOSTIMULINE

(30) Priorität: 21.07.2000 DE 10035706
(43) Veröffentlichungstag der Anmeldung: 16.04.2003
(73) Patentinhaber: B.R.A.H.M.S Aktiengesellschaft, 16761 Hennigsdorf (DE)
(72) Erfinder: COSTAGLIOLA, Sabine, B-1080 Brüssel (BE); VASSART, Gilbert, B-1200 Brüssel (BE); BERGMANN, Andreas, 12351 Berlin (DE); STRUCK, Joachim, 12161 Berlin (DE); MORGENTHALER, Nils, G., 13503 Berlin (DE)
(74) Vertreter: Andrae, Steffen, Dr.
(86) Internationale Anmeldenummer: PCT/EP2001/008252
(87) Internationale Veröffentlichungsnummer: WO 2002/008723

(56) Entgegenhaltungen:
- EP-A- 0 298 368
- EP-A- 0 414 224
- WO-A-98/20343
- WO-A-98/26294
- DE-C- 19 907 094
- MORGENTHALER N G: "NEW ASSAY SYSTEMS FOR THYROTROPIN RECEPTOR ANTIBODIES" CURRENT OPINION IN ENDOCRINOLOGYAND DIABETES, PHILADELPHIA, PA, US, Bd. 6, Nr. 4, 1999, Seiten 251-260, XP000944171 ISSN: 1068-3097 in der Anmeldung erwähnt

## Beschreibung

Die vorliegende Erfindung betrifft ein Verfahren zur selektiven Bestimmung eines ausgewählten Subtypen von Autoantikörpern gegen den TSH-Rezeptor (TRAb), nämlich sogenannten blockierenden TRAb (TBAb).

Der Rezeptor für das Hormon TSH (thyroidstimulierendes Hormon, Thyr(e)otropin), der TSH-Rezeptor (TSH-R), spielt eine Schlüsselrolle für die Funktion und das Wachstum von Schilddrüsenzellen. Dieser Rezeptor ist ein Glied einer Unterfamilie der G-Protein-gekoppelten Glykoproteinrezeptoren, die außerdem insbesondere noch die Rezeptoren für das luteinisierende Hormon/Choriongonadotrophin (LH/CG-R) und das follikelstimulierende Hormon (FSH-R) umfaßt. Die Rezeptoren dieser Unterfamilie weisen eine große N-terminale extrazelluläre Domäne auf, die für die Ligandenbindung von essentieller Bedeutung ist und für die gezeigt wurde, daß sie an der Signalübermittlung beteiligt ist. Die Übermittlung des TSH-R-Signals wird überwiegend durch die Aktivierung von Adenylatcyclase vermittelt, was zu einer Erhöhung des intrazellulären cAMP-Niveaus führt.

Ein Teil des großen Interesses an dem TSH-R ist auf seine Rolle als primäres Autoantigen bei verschiedenen Schilddrüsen-Autoimmunerkrankungen zurückzuführen, die vom Auftreten von Autoantikörpern gegen den TSH-R begleitet sind (TSH-R-Auto-Ab bzw. nachfolgend stets nur kurz TRAb). Zu derartigen Schilddrüsen-Autoimmunerkrankungen gehören insbesondere die Basedowsche Krankheit (Morbus Basedow; engl.: Graves' disease), eine zu Schilddrüsenüberfunktion führende Autoimmunerkrankung, die zu den häufigsten menschlichen Autoimmunerkrankungen überhaupt gehört, sowie die Hashimoto Thyreoiditis und das idiopathische Myxödem, die mit einer Schilddrüsenunterfunktion verbunden sein können.

Der TSH-R kommt in der Oberfläche von Schilddrüsenzellen nur in sehr geringen Mengen (in der Größenordnung von 1.000 - 10.000 Rezeptoren pro Zelle) vor. Eine Aufklärung der chemischen Struktur des humanen TSH-Rezeptors (hTSH-R), d.h. der Sequenz der für ihn codierenden DNA sowie der daraus ableitbaren Aminosäuresequenz des Rezeptors selbst, gelang Ende 1989 (vgl. Libert F. et al., Biochem. Biophys. Res. Commun. 165: 1250-1255; Nagayama Y. et al., Biochem. Biophys. Res. Commun. 165: 1184-1190; vgl. auch EP-A-0433509 bzw. WO-A-91/09121; sowie WO-A-91/09137; WO-A-91/10735 und WO-A-91/03483; ferner Yuji Nagayama & Basil Rapoport, in: Molecular Endocrinology, Vol. 6 No. 2, S. 145-156 und die darin zitierte Literatur) . Seitdem wurden das hTSH-R-Polypeptid und ausgewählte Teilpeptide davon in einer Vielzahl von Systemen exprimiert, wobei sich zeigte, daß ein funktionaler TSH-R nur in Säugetierzellen hergestellt werden kann, und zwar sowohl transient als auch in Form einer stabilen Zellinie, z.B. unter Verwendung von Adenovirus- und Vacciniavirus-Expressionssystemen.

Das erfolgreiche molekulare Klonieren des hTSH-R hat es ermöglicht, neue Einsichten in die Wechselwirkung von TSH sowie TRAb mit dem TSH-R zu gewinnen und diese Erkenntnisse auch bei der Bestimmung von TRAb für diagnostische Zwecke zu nutzen. Ein Überblick über den gegenwärtigen Stand der Bestimmung von TRAb im Licht der neueren wissenschaftlichen Erkenntnisse findet sich im Artikel von Nils G. Morgenthaler: New assay systems for thyrotropin receptor antibodies; in Curr Opin Endocrinol 1999, 6:251-260. In der vorliegenden Anmeldungen werden für Analyten und Reagenzien die in dem genannten Artikel definierten Abkürzungen und Bezeichnungen verwendet. Auf den Inhalt des genannten Artikels sowie die darin genannten Literaturstellen wird zur Ergänzung des Offenbarungsgehalts der vorliegenden Anmeldung ausdrücklich verwiesen.

Wie auch in dem genannten Artikel zusammenfassend ausgeführt wird, ist der einzige gegenwärtig für den klinischen Alltag verwendbare Assay zur Bestimmung von TRAb ein indirekt arbeitender kompetitiver Assay, bei dem die auf die Anwesenheit von TRAb in einer Patientenprobe zurückzuführende Inhibierung der Bindung von markiertem bTSH an einen solubilisierten porcinen TSH-R aus Schweine-Schilddrüsen (traditioneller Assayaufbau) oder, im Falle eines Assays der neuen "zweiten Generation", an einen an eine Festphase, z.B. Coated Tubes, immobilisierten rekombinanten humanen TSH-R (rhTSH-R) zur Messung genutzt wird. Sich von den traditionellen Assays unterscheidende neuartige Festphasen-Assays sind beschrieben in DE 196 51 093 oder WO 98/26294 bzw. DE 198 01 319 oder WO 99/3678 bzw. in J.Clin.Endocinol. Metab., Vol.84, No.1, S.90-97 (1999). Aufgrund des angewandten Meßprinzips erfaßt ein solcher Assay nur mit bTSH kompetierende Autoantikörper vom sogenannten TBII-Typ, ohne zwischen einzelnen Subtypen (stimulierend, blokkierend) zu unterscheiden.

Insbesondere in Patentanmeldungen, die verfahrensvarianten von Assays zur Bestimmung von TRAb vom TBII-Typ betreffen, wird in allgemeiner Form auch davon gesprochen, daß man statt der Inhibierung von TSH auch die Inhibierung von Antikörpern gegen den TSH-R zur Messung nutzen kann, insbesondere auch zusätzlich zu einer Inhibierung von TSH, wie z.B. in DE 196 51 093.7 bzw. WO 98/26294 beschrieben wird. In allen derartigen Fällen geht es jedoch stets um eine möglichst vollständige Erfassung aller relevanten pathogenen TRAb in einer Patientenprobe, um auf diese Weise auf Autoimmunreaktionen zurückzuführende Schilddrüsenerkrankungen möglichst lückenlos zu erkennen, ohne daß patientenbedingte Unterschiede der Zusammensetzung der individuell auftretenden TRAb-Populationen zu falsch negativen Meßergebnissen führen, d.h. es geht um eine Erhöhung der klinischen Relevanz von TRAb-Bestimmungen bei kompetitiven Assays.

Es ist inzwischen bekannt, daß die bei Autoimmunerkrankungen auftretenden TRAb auch im Falle von Morbus Basedow Patienten nicht nur stimulierende Autoantikörper (TSAb), die zu einer Hyperthyreose führen, umfassen, sondern daß daneben möglicherweise auch noch blockierende Autoantikörper (TBAb) vorkommen. Die bekannten kompetitiven Assays können nicht zwischen TSAb und TBAb unterscheiden, sondern erfassen diese unterschiedslos als TBII.

Will man zwischen TSAb und TBAb unterscheiden, was im Sinne einer Feindiagnostik des Autoimmungeschehens z.B. bei Morbus Basedow von großem wissenschaftlichem und letztlich auch klinischem Interesse sein kann, muß man auf Bioassays zurückgreifen, auf die z.B. auch in dem o.g. Artikel von N. Morgenthaler in: Curr.Opin.Endocrinol. 1999, 6:251-260 näher eingegangen wird. Bei solchen Bioassays wird ermittelt, ob die cAMP-Produktion von Zellen, die in ihrer Membran einen natürlichen oder rekombinanten TSH-R enthalten, durch die Anwesenheit von bestimmten Seren bzw. Autoantikörpern erhöht wird bzw. ob die durch eine gegebene TSH-Konzentration in der Zelle ausgelöste cAMP-Produktion durch die Seren oder Autoantikörper geschwächt wird.

Da Bioassays für alltägliche Untersuchungen von Patientenproben in Klinik und Labor zu aufwendig sind, wäre es allerdings wünschenswert, auch Assays zur Verfügung zu haben, die eine Unterscheidung von pathogenen stimulierenden und blockierenden TRAb auf einfachere Weise ermöglichen. Ein Vorschlag für einen derartigen Assay, der Immunpräzipitation bzw. darauf beruhende Differenzmessungen nutzt, findet sich in den Patentschritten DE 199 49 184 bzw. DE 100 07 792.

Es ist Aufgabe der vorliegenden Erfindung, ein differentialdiagnostisches Verfahren zur selektiven Bestimmung von bestimmten TRAb, und zwar von TBAb, zu schaffen, bei dem im wesentlichen die eingeführten Arbeitsweisen zur TBII-Bestimmung zur Anwendung kommen können und das sich daher auch für die klinische Praxis eignet.

Diese Aufgabe wird in allgemeiner Form durch ein Verfahren gemäß Anspruch 1 gelöst.

Vorteilhafte, gegenwärtig bevorzugte Ausgestaltungen des Verfahrens gemäß Anspruch 1 finden sich in den Unteransprüchen 2 bis 12 sowie der nachfolgenden Beschreibung unter Berücksichtigung der Ausführungsbeispiele.

Im vorausgehenden und nachfolgenden Text dieser Anmeldung werden die verwendeten Reagenzien bzw. Analyten/Biomoleküle in der Regel durch verschiedene Abkürzungen gekennzeichnet, die stets in den folgenden Bedeutungen zu verstehen sind, es sei denn, es ergibt sich für den Fachmann aus dem konkreten Zusammenhang etwas anderes. Die Verwendung der speziellen Angaben erfolgt dabei aus Gründen der exakten Beschreibung der durchgeführten Versuche und Messungen, bedeutet jedoch nicht, daß die beschriebenen Ergebnisse und Schlußfolgerungen nur für den beschriebenen Spezialfall gelten und nicht unter Heranziehung des relevanten Fachwissens verallgemeinert werden können.
- TSH =: Thyroidstimulierendes Hormon (Thyreotropin). Wird die Abkürzung TSH ohne weitere Zusätze verwendet, handelt es sich nicht um ein bestimmtes Produkt, sondern die Bindung bzw. Funktion des Hormons wird in allgemeiner Form diskutiert.
- ¹²⁵I -bTSH =: Radioiodiertes, als Kompetitor eingesetztes bTSH (bovines TSH). Im Zusammenhang mit der Beschreibung von Assays der Firma B.R.A.H.M.S Diagnostica GmbH steht ¹²⁵I-bTSH insbesondere für ein Produkt, wie es gemäß DE 42 37 430 C1 bzw. EP 0 668 775 B1 erhalten wird und Bestandteil des TRAK-Assay® bzw. des DYNOtest® TRAK human der Fa. B.R.A.H.M.S Diagnostica GmbH ist.
- TSH-R =: Der TSH-Rezeptor, ein in der Schilddrüsenmembran verankerter Glykoproteinrezeptor. Wird die Abkürzung TSH-R ohne weitere Zusätze verwendet, ist keine Beschränkung auf irgendein bestimmtes Produkt beabsichtigt: Es kann sich somit auch um einen aus natürlichem Material gewonnenen, z.B. porcinen TSH-R handeln. Bei einem in einem erfindungsgemäßen Assay verwendeten TSH-R handelt es sich jedoch vorzugsweise um ein gentechnisch erzeugtes (rekombinantes) funktionales Rezeptor-Polypeptid, das die Aminosäuresequenz und ggf. Glykosylierung irgendeines natürlich vorkommenden TSH-R, besonders bevorzugt eines humanen TSH-R (hTSH-R), wenigstens in einem solchen Ausmaße aufweist, daß es als "funktionaler TSH-Rezeptor" bezeichnet werden kann. Die Charakterisierung "funktional" bedeutet, daß es sich bezüglich der Bindung von TRAb in signifikantem Ausmaß wie der natürlich vorkommende, insbesondere humane, TSH-R verhält. Die Abkürzung TSH-R kann dabei für das rekombinante vollständige, mehr oder weniger stark glykosylierte Polypeptid, eine Teilsequenz einer ausreichenden Länge oder ein gentechnologisch erzeugtes Fusionsprodukt davon stehen. Ohne weitere Erläuterungen liegt ein einfach als TSH-R bezeichnetes Produkt als Membranpräparation vor, aus der das Produkt ggf. durch geeignete Solubilisierung der Membranen der zur Expression des rekombinanten Polypeptids verwendeten Zellen unter Verwendung von Detergenzien auch als aufgereinigter Extrakt erhalten worden sein kann.
- TRAb =: In biologischen Proben, insbesondere humanem Serum oder Plasma, nachweisbare Autoantikörper gegen den TSH-Rezeptor (TSH-R-Auto-Ab) beliebiger Spezifität.
- TSAb =: Die Schilddrüse stimulierende TRAb. Ihr Nachweis ist insbesondere für die Diagnose des Morbus Basedow (englisch: Graves' disease) von Bedeutung.
- TBAb =: Die Wirkung von TSH (sowie ggf. auch von TSAb) auf die Schilddrüse blockierende TRAb, deren Nachweis zu einer Hypothyreose in Beziehung gesetzt werden kann.
- TBII =: In kompetitiven Assays, die auf einer Konkur- renz von markiertem TSH mit TRAb um die Bindungsstellen einer TSH-R-Präparation beruhen, erfaßbare TRAb. Sie können TSAb und/oder TBAb und/oder neutrale TRAb sein. Kommerzielle Assays zur Bestimmung von TRAb vom TBII-Typ sind die Assays TRAK-Assay® und DYNOtest® TRAK human (Radiorezeptorassays) bzw. LUMItest® TRAK human (Chemilumineszenzrezeptorassay) der Fa. B.R.A.H.M.S Diagnostica GmbH.
- anti-TSH-R-Ab =: In erfindungsgemäßen Assays als selektiver Kompetitor (erstes Immunreagens) eingesetzte, gegebenenfalls markierte oder selektive markierbare Antikörper gegen den TSH-R. Die für das Verfahren benötigte Selektivität dieser Antikörper wird in erster Linie bei Verwendung selektiver monoklonaler Antikörper (anti-TSH-R-moAb) gewährleistet, deren Herstellung und Selektion im experimentellen Teil beschrieben ist. Die als Immunreagens eingesetzten anti-TSH-R-Ab sind von den als TRAb abgekürzten Autoantikörpern aus Patientenproben klar zu unterscheiden.
- moAb =: monoklonale Antikörper.

Nachfolgend wird das erfindungsgemäße Verfahren noch näher erläutert. Im experimentellen Teil wird die Herstellung von blockierenden monoklonalen Antikörpern sowie ihre Verwendung zur Bestimmung der Menge blockierender Autoantikörper in Patientenseren anhand einer konkreten Arbeitsvorschrift und eines Ausführungsbeispiels genauer beschrieben. In den Figuren zeigen:
- Fig. 1: die Ergebnisse der Versuche zur Charakterisierung von 12 moAb, bei denen man zur Messung ihrer TBII-Aktivität die selektierten moAb mit markiertem bTSH um Bindungsstellen eines TSH-R, eingesetzt in Form von JP09 Zellen, kompetieren ließ;
- Fig. 2: die Ergebnisse der Untersuchung der gleichen moAb, wie in Fig. 1 in einem Bioassay, in dem die blokkierenden Eigenschaften der moAb im Sinne einer Verminderung der Stimulation der cAMP-Produktion in JP09-Zellen durch TSH bestimmt wurde;
- Fig. 3: die Bestimmung des Bindungsverhaltens der moAb an TSH-R-Chimären, die von COS-Zellen exprimiert werden, und zwar zum Zwecke der Feststellung des Ortes des von dem blockierenden moAb erkannten Epitops auf der extrazellulären Domäne (ECD) des TSH-R;
- Fig. 4: die Ergebnisse der Messung von Seren von Morbos Basedow Patienten unter Verwendung von JP19-Zellen als TSH-R-Immunreagens und unter Verwendung eines ¹²⁵I-moAb (moAb 23.1) als Kompetitor.

Geht man von bekannten Verfahren aus, die auf der Bestimmung von TRAb vom TBII-Typ aus Patientenseren durch Kompetition mit markiertem bTSH um die Bindungsstellen eines TSH-R-Präparats beruhen, d.h. von Assays, wie sie in den Assays TRAK-Assay® und DYNOtest® TRAK human bzw. LUMItest® TRAK human der B.R.A.H.M.S Diagnostica GmbH verkörpert sind, kann man das erfindungsgemäße Verfahren vereinfacht so darstellen, daß bei dem erfindungsgemäßen Verfahren als Kompetitor anstelle des markierten bTSH ein selektiver markierter Kompetitor verwendet wird, der im wesentlichen nur mit einer engen, eindeutig definierten Untergruppe von TRAb aus dem Patientenserum, nämlich Nicht-TSAb bzw. TBAb, kompetiert und dadurch die selektive Bestimmung von TBAb ermöglicht.

Vorzugsweise wird das erfindungsgemäße Verfahren dabei in einer Verfahrensvariante als Festphasen-Assay durchgeführt, der in vieler Beziehung den Assays zur TRAb-Bestimmung der "zweiten Generation" (DYNOtest® TRAK human; LUMItest® TRAK human; vgl. die jeweiligen Gebrauchsanweisungen der B.R.A.H.M.S Diagnostica GmbH) entspricht. Bei dieser Verfahrensvariante ist oder wird das als selektiver Binder eingesetzte Immunreagens, d.h. die TSH-R-Präparation, insbesondere eine rhTSH-R-Präparation, mit Hilfe eines speziellen monoklonalen Antikörpers (BA8; vgl. J. Clin. Endocrinol Metab 84: 90-97, 1999 bzw. J.Immunol. 160: 1458-1465) immobilisiert, während der Kompetitor markiert ist. Es können einerseits alle bei den bekannten Verfahren zur Markierung von bTSH verwendeten Markierungen (¹²⁵I; Chemilumineszenzlabel, z.B. in Form eines Akridiniumderivats) verwendet werden, es können bei dem erfindungsgemäßen Verfahren jedoch auch andere an sich bekannte Markierungslabel zum Einsatz kommen, z.B. Enzymlabel oder Fluoreszenzlabel, die an Immunreagenzien vom Antikörpertyp leichter ohne Aktivitätsverlust des Reagens angefügt werden können als zum Beispiel an TSH.

Es können außerdem auch an sich bekannte Nachweissysteme zur Anwendung kommen, bei denen an das als Kompetitor eingesetzte Immunreagens eine erste Markierungskomponente gebunden ist, die Teil eines komplexen Nachweissystems ist, dessen anderer Teil an ein weiteres Immunreagens, z.B. einen weiteren Antikörper, gebunden ist. Beim erfindungsgemäßen Verfahren kann der weitere, mit der zweiten Markierungskomponente verknüpfte Antikörper zum Beispiel ein Antikörper sein, wie er bei den TBII-Assays der zweiten Generation zur Immobilisierung des hTSH-R-Präparats verwendet wird. Bei der Ausbildung eines Sandwich unter Beteiligung der beiden Antikörper kommt es zu einer charakteristischen Änderung eines Meßsignals, z.B. eines Fluoreszenz- oder Chemilumineszenz-Signals, die das Ausmaß der Bindung beider Antikörper an den Rezeptor und damit indirekt die Konzentration eines gesuchten Analyten widerspiegelt. Als Beispiel für ein solches System können die z.B. in US-A-4,822,733, EP-0-180 492 B1 oder EP-B1-0 539 477 und dem darin zitierten Stand der Technik beschriebenen Markierungssysteme genannt werden.

Wenn man auf ein komplexes Markierungssystem der genannten Art zurückgreift, kann der Assay auch als homogener Assay ausgestaltet sein, bei dem sich alle Immunreagenzien, Analyten und Reaktionsprodukte in einer flüssigen Reaktionsmischung befinden bzw. bilden und bei dem die Messung, die selektiv nur Reaktionsprodukte erfaßt, die beide Markierungskomponenten in einem einzigen Immunkomplex enthalten, direkt in der Reaktiosmischung erfolgt. Als Beispiel ist wiederum auf die drei obigen Patentveröffentlichungen, den darin genannten Stand der Technik sowie die unter den Marken TRACE® (Time Resolved Amplified Cryptate Emission) bzw. KRYPTOR® angebotetene Technologie (vgl. Prospekte der CIS Diagnostik GmbH) zu verweisen.

Im Falle von Festphasen-Assays kann die Immobilisierung des TSH-R kann an eine beliebige Festphase erfolgen, beispielsweise Polystyrolröhrchen, die als "Coated Tubes" verwendet werden, oder an Mikrotiterplatten, zum Beispiel aus Polystyrol, oder an Partikel, beispielsweise Magnetpartikel. Auch eine zeitlich verzögerte bzw. nachträgliche Immobilisierung unter Verwendung geeigneter Festphasen ist möglich.

Der TSH-R kann bei dem erfindungsgemäßen Verfahren auch auf andere Weise als mit Hilfe spezieller Antikörper immobilisiert werden. Setzt man beispielsweise ein TSH-R-Fusionsprodukt ein, bei dem der TSH-Rezeptor oder der für die gewünschte Immunreaktion erforderliche Teil davon mit Nichtrezeptor-Anteilen, beispielsweise einem biotinylierten Rest, einem sog. 6His-tag aus 6-Histidinresten oder einem anderem geeigneten Rest (vgl. z.B. WO 98/20343), der zur Immobilisierung eine Reaktion mit einem an einer Festphase befindlichen Reaktionspartner, z.B. Streptavidin, Nickel-Chelate oder einem für den Rest spezifischen Antikörper, eingehen kann, können auch derartige Immobilisierungssysteme für den TSH-R verwendet werden, vorausgesetzt, daß dieser seine für die kompetierenden Immunreaktionen erforderliche Funktionalität bewahrt und das Immobilisierungssystem diese Immunreaktionen nicht behindert.

Als festphasenassay kann das Verfahren auch in einer als "spiegelbildlich" bezeichenbaren Ausführungsform durchgeführt werden, bei der das als Kompetitor verwendete Immunreagens, d.h. beispielsweise der blockierende monoklonale Antikörper, immobilisiert ist und mit einem markierten solubilisierten TSH-Rezeptor gearbeitet wird, beispielsweise einem, wie er in DE 198 01 154 beschrieben ist bzw. wie er nach der Lehre der genannten Patentanmeldung erhältlich ist. Die Kompetition des immobilisierten Kompetitors mit den TRAb aus den Patientenproben äußert sich auch in diesem Falle als Verminderung der Bindung des verwendeten Markers an die Festphase. Derartige spiegelbildliche Assay-Varianten werden in den oben genannten Patentanmeldungen der Anmelderin diskutiert, kommen jedoch bisher in kommerziellen Assays zur TRAb-Bestimmung nicht zur praktischen Anwendung.

Während somit das Assay-Grunddesign auf an sich bekannte Weise in Analogie zu Assays, die mit markiertem bTSH arbeiten, vielfältig variiert werden kann, beispielsweise bezüglich des verwendeten Markierungssystems, der verwendeten Festphase sowie der Wahl des immobilisierten und des markierten Immunreagens, oder der Assay auch im obengenannten Sinne als homogener Assay ausgestaltet sein kann, ohne daß der Bereich des erfindungsgemäßen Verfahrens verlassen wird, ist es für das erfindungsgemäße Verfahren zwingend, daß das Immunreagens, das als Kompetitor für die zu bestimmenden TRAb dient, nicht ein markiertes bTSH ist, sondern ein Immunreagens mit einer hohen, definierten Spezifität, die es gewährleistet, daß das Immunreagens selektiv mit einem definierten Typ von TRAb, nämlich blockierenden TRAb (= TBAb), kompetiert.

Um ein solches Verfahren verwirklichen zu können, ist es somit Voraussetzung, ein derartiges selektives Immunreagens einer definierten Reaktivität zur Verfügung zu haben, das nicht nur mit TSH, sondern auch mit den zu bestimmenden TRAb kompetiert, d.h. daß es auch die dafür erforderliche Affinität aufweisen muß.

Im nachfolgenden experimentellen Teil wird erstmals gezeigt, daß ein derartiges spezifisches Immunreagens in Form eines monoklonalen Antikörpers (Anti-TSH-R-moAb) nach einem detailliert beschriebenen Verfahren herstellbar ist und in der Lage ist, mit TBAb aus Patientenseren um gemeinsame Bindungsstellen eines rekombinanten hTSH-R, genauer der extrazellulären Domäne eines rhTSH-R, zu kompetieren.

Blockierende Anti-TSH-R-moAb mit den gewünschten Eigenschaften können, wie nachfolgend beschrieben, im Anschluß an eine genetische Immunisierung nach einem Verfahren erhalten werden, wie es in seinen Grundzügen beschrieben ist in J.Immunol. 160: 1458-1465.

Bei diesem Verfahren wird ein geeignetes ausgewähltes Versuchstier (Maus) mit einem cDNA-Konstrukt, das die cDNA für die extrazelluläre Domäne (ECD) des hTSH-R enthält, immunisiert. Nach einer Boosterinjektion entsprechend einem empirischen Immunisierungsplan werden Seren aus den Versuchstieren gewonnen, und durch FACS (fluorescence-activated cell sorter) Analyse unter Verwendung von Zellen, beispielsweise transformierten CHO-Zellen, die den hTSH-R exprimieren (JP09), sowie Antimaus-IgG zur Fluoreszenzmarkierung, können dann diejenigen Mäuse selektiert werden, bei denen es zur Bildung von Antikörpern mit der gesuchten Reaktivität gegen des TSH-R gekommen ist. Die Stärke der Antikörperbildung kann durch Kompetition der Mäuse-Seren mit ¹²⁵I-bTSH um einen von tranformierten Zellen exprimierten rekombinanten hTSH-R bestimmt werden. Außerdem können in einem parallelen Bioassay die Seren darauf untersucht werden, ob sie die cAMP-Produktion in den TSH-R exprimierenden Zellen mehr oder weniger stark fördern oder blockieren.

Hat man eine Maus identifiziert, die nach Maßgabe der Testung ihres Serums eine Antikörperproduktion der gewünschten Art zeigt, kann eine solche Maus nach der an sich bekannten Technik zur Erzeugung von moAb herangezogen werden, indem die Splenozyten der Maus mit Myelom-Zellen fusioniert werden, die gebildeten Hybridome gezüchtet werden und nach dem Verdünnungsprinzip für die Produktion von moAb herangezogen werden.

Die Eigenschaften der erzeugten moAb können im wesentlichen analog wie die Eigenschaften der Seren festgestellt werden, so daß moAb mit den gewünschten Eigenschaften selektiert werden können.

Wie im nachfolgenden experimentellen Teil beschrieben wird, wurde dabei einer der erzeugten moAb, der stark blockierende Eigenschaften zeigte, für Kompetitionsversuche ausgewählt und zu diesem Zweck markiert. Es konnte gezeigt werden, daß er mit TRAb aus Patientenseren selektiv kompetiert.

Um zu ermitteln, wo das Epitop für die Bindung des genannten moAb auf der extrazellularen Domäne (ECD) des TSH-R angeordnet ist, wurden für diese Untersuchung ferner geeignete Chimären konstruiert, und anhand einer auftretenden oder ausbleibenden Bindung des markierten moAb an die jeweilige Chimäre konnte das Epitop, um das die untersuchten moAb (Anti-TSH-R-Ab) mit den TRAb aus der Patientenprobe konkurrieren, lokalisiert werden. Im beschriebenen Fall lag das Epitop im Bereich der Aminosäuren 382 bis 415 der bekannten Sequenz des hTSH-R.

Nachfolgend wird die Erfindung anhand der erfolgreichen Experimente zur moAb-Herstellung und -Selektion sowie anhand der mit einem der selektierten moAb durchgeführten Kompetitionsversuchen näher erläutert.

### Materialien und Methoden -Ergebnisse

### 1. Produktion und Charakterisierung von monoklonalen Antikörpern (anti-TSH-R-moAb)

### 1a. Immunisierung von Versuchstieren und Gewinnung von Blutproben

Im Unterschied zu den früheren Untersuchungen zur genetischen Immunisierung von Mäusen (J.Immunol. 160:1458-1465, 1998) wurden für die folgenden Immunisierungsversuche 6 Wochen alte weibliche NMRI-Mäuse [ico:NMRI (IOPS:Han)] eingesetzt. Die Mäuse waren ursprünglich Abkömmlinge von Schweizer Mäusen und wurden unter Auszuchtbedingungen im Institut für Versuchstierkunde am Zentralen Labor der TU Hannover, Deutschland gehalten. Die Mäuse tolerieren keine gegenseitigen Hautverpflanzungen. Sie wurden unter den vorgeschriebenen Tierschutzbedingungen gehalten und behandelt.

Am Tag 0 wurden ihnen unter Nembutal-Anästhesie 100 µg pcDNAIII-hTSH-R in PBS (vgl. J.Immunol. 160:1458-1465, 1998) in den anterioren Schienbeinmuskel injiziert. In den Muskel waren 5 Tage vorher 100 µl Cardiotoxin (10 mM, aus dem Gift der Naja nigricollis gereinigt, Calbiochem, La Jolla, CA) injiziert worden. Die Injektionen wurden 4 Wochen und 8 Wochen danach jeweils wiederholt. In der 12. Woche nach der ersten Immunisierung wurden den immunisierten Mäusen Blutproben aus den Retrooculär-Kapillaren des rechten Auges entnommen. Für alle Bestimmungen wurden die jeweiligen, so gewonnenen Seren individuell untersucht.

### 1b. Seren- und Hybridom-Überstands-Untersuchungen durch FACS-Analyse

Transformierte CHO-Zellen, die den hTSH-R exprimieren (JP09; vgl. Biochem.Biophys.Res.Commun., 1990, Vol.171, S.1044-1050) oder Kontroll-CHO-Zellen (JP02) wurden von den Platten, auf denen sie gezüchtet wurden, mit einer EDTA und EGTA (jeweils 5 mM) enthaltenden Phosphat-gepufferten Salzlösung (PBS-Lösung) abgelöst und in Falcon 2052-Röhrchen (200.000 Zellen pro Röhrchen) überführt. Die Zellen wurden bei 500 g bei 4°C für 3 min zentrifugiert, und der Überstand wurde durch Inversion entfernt. Anschließend wurden die Röhrchen 30 min bei Raumtemperatur mit 100 µl PBS-BSA, 0,1%, mit einem Gehalt von 2 µl Serum oder, im Falle der Prüfung auf moAbs, 10 µl KulturÜberstand von Hybridomzellen inkubiert. Die Zellen wurden dann mit 4 ml PBS-BSA, 0,1%, gewaschen und wie oben zentrifugiert. Sie wurden 30 min auf Eis in der Dunkelheit mit einem Fluorescein-konjugierten Gamma-Ketten-spezifischen Ziegen-Anti-Maus-IgG (SIGMA, St. Louis, MO) im gleichen Puffer inkubiert. Zur Erkennung von beschädigten Zellen, die von der Analyse ausgeschlossen wurden, wurde Propidiumjodid (10 µg/ml) verwendet. Die Zellen wurden noch einmal gewaschen und in 250 µl PBS-BSA, 0,1% resuspendiert. Die Fluoreszenz von 5000 Zellen pro Röhrchen wurde mit Hilfe eines FACScan® Flow Cytofluorometer (Becton Dickinson, Eerenbodegem, Belgien) untersucht.

### 1c. Messungen der TBII-Aktivität der Mäuseseren

Zur Messung der TBII-Aktivität wurden intakte CHO-Zellen (JP09, s.o.), die den hTSH-R exprimieren, verwendet. Zu diesem Zwecke wurden auf Mikrotiterplatten mit 96 Vertiefungen 5 x 10⁴ Zellen/Vertiefung in 0,1 ml modifiziertem Hank's Puffer ohne NaCl (die Isotonie wurde mit 280 mM Saccharose gewährleistet), ergänzt mit 2,5% Magermilch, ¹²⁵I-bTSH (30.000 cpm; Tracer aus dem TRAK-Assay® der B.R.A.H.M.S Diagnostica GmbH) und Mäuseserum 4 h bei Raumtemperatur inkubiert. Bei der Beendigung der Inkubation wurden die Zellen rasch im gleichen eiskalten Puffer gewaschen und mit 0,2 ml 1 N NaOH solubilisiert, und die Radioaktivität wurde in einem Gamma-Counter gemessen. Dabei wurden zur Messung der TBII-Aktivität 3 µl Mäuseserum pro Vertiefung verwendet. Alle Versuche wurden dreifach wiederholt, und die Ergebnisse werden als gebundene CPM (counts per minute) ausgedrückt.

Bei der Prüfung der Eigenschaften von moAbs wurden statt der Mäuseseren Hybridom-Kulturüberstände eingesetzt.

### 1d. Messungen der TSAb- und TBAb-Aktivität der Mäuseseren

Bei dem Bioassay zur Bestimmung der TSAb- bzw. TBAb-Aktivität wurden wiederum transformierte CHO-Zellen verwendet, die den hTSH-R exprimieren (JP26; Biochem. Biophys. Res. Commun. 171: 1044-1050, 1990) verwendet. Kurz gesagt wurden auf Mikrotiterplatten mit 96 Vertiefungen 5 x 10⁴ Zellen/Vertiefung in 5 mM KCl, 0,25 mM KH₂PO₄, 0,5 mM MgSO₄, 0,4 mM Na₂HPO₄, 1 mM CaCl₂, 0,1% Glucose, 2 mM IBMX, 20 mM Hepes und 0,3% BSA mit einer Menge von 3 µl Serum (Gesamtvolumen 100 µl pro Vertiefung) inkubiert. Die Inkubation erfolgte für 4 h bei 37°C. Das in das Medium freigesetzte cAMP wurde unter Verwendung eines kommerziellen kompetitiven Bindungsassays auf an sich bekannte Weise gemessen (Amersham, Bucks, U.K.). Dabei wurde die TSAb-Aktivität unter den obigen Grundbedingungen gemessen, während zur Messung einer TBAb-Aktivität unter identischen Bedingungen und unter Zugabe von 200 µU/ml Endkonzentration von bTSH (SIGMA) gearbeitet wurde. Bei allen Versuchen wurden Duplikatproben untersucht, und die Ergebnisse werden als pmol cAMP/ml ausgedrückt.

Bei der Prüfung der Eigenschaften von moAbs wurden statt der Mäuseseren Hybridom-Kulturüberstände eingesetzt.

### 1e. Erzeugung und Selektion von monoklonalen Antikörpern

Zur Isolierung monoklonaler Antikörper wurden ausgewählte Mäuse, die bei der oben beschriebenen Analyse ihrer Seren die gewünschte Immunreaktion zeigten, durch intravenöse Injektion von 5 µg ECD-6H-GPI (einem ECD-GPI-Fusionsprotein mit einem 6-Histidin-Tag, der zwischen die ECD und das Signalpeptid, das den GPI-Anker kontrolliert, inseriert war) geboostert.

Dabei wurden zur Gewinnung der injizierten Flüssigkeit transformierte CHO-Zellen, die einen hohen Anteil an ECD-6H-GPI-Protein (Zelllinie 4648) exprimieren, 3 h mit PI-PLC inkubiert. Das flüssige Medium wurde anschließend entfernt und mit einem Kobaltharz zur Bindung des 6-Histidin-Tags 1 h bei 4°C inkubiert (Talon^{TM} Metal Affinity Resin, CLONTECH Laboratories, Inc., Palo Alto, USA). Das Harz wurde mit einem Tris-NaCl-Puffer, pH 8, der 10 mM Imidazol enthielt, gewaschen. Anschließend wurden die gebundenen Proteine mit 100 mM Imidazol von dem Harz eluiert.

3 Tage nach der beschriebenen Booster-Injektion wurden Splenozyten einer ausgewählten NMRI-Maus (vgl. nachfolgend unter 3.), die wie unter 1a. beschrieben mit pcDNAIII-TSH-R immunisiert worden war, mit SP2/0 Myelomzellen bei einem Verhältnis von 5:1 unter Verwendung von 50% Polyethylenglykol verschmolzen. Nach der Verschmelzung wurden die Zellen in Mikrotiterplatten mit 96 Vertiefungen in einer Dichte von 2 x 10⁵ Zellen/Vertiefung in einem selektiven Medium ausgesät (Dulbecco's Modified Eagle's Medium, das 10% fötales Kälberserum, 2% Nutridoma (Boehringer Mannheim, Belgien), 10 mM Natriumpyruvat, 2 mM L-Glutamin, 5 mM β-Mercaptoethanol, 2,5 U/ml Amphotericin B, 100 U/ml Penicillin, 100 µg/ml Streptomycin, 100 µM Hypoxanthin, 400 µM Aminopterin, 16 µM Thymidin enthielt). 10 Tage nach der Verschmelzung, während die Zellen aktives Wachstum zeigten, wurden die Kulturüberstände wie oben unter 1b. beschrieben durch FACS-Analyse mit JP09-Zellen auf Anti-hTSH-R-Antikörper untersucht. Hybridomzellen, deren Überstände die gewünschte Immunreaktivität zeigten, wurden durch limitierende Verdünnung kloniert. Hybridoma-Klone wurden weiter als Aszites in Pristan-vorbehandelten BALB/c-Mäusen gezüchtet. Zur Reinigung von IgG aus der Mäuse-Aszites-Flüssigkeit wurde eine Sepharose-Protein A-Affinitäts-Chromatographie angewandt. Die Ig-Klasse der moAb wurde mit einem Mäuse-moAb-Isotypier-Kit (IsoStrip™, Boehringer Mannheim, Belgien) bestimmt.

Für die nachfolgend beschriebenen Kompetierungsversuche wurde ein mAb, und zwar der moAb 23.1, nach der Chloramin-T-Methode so iodiert, daß seine spezifische Radioaktivität 80 µCi/µg betrug.

### 1f. Ermittlung der Bindungsstellen ausgewählter moAbs bzw. damit kompetierender TRAb auf der ECD eines hTSH-R

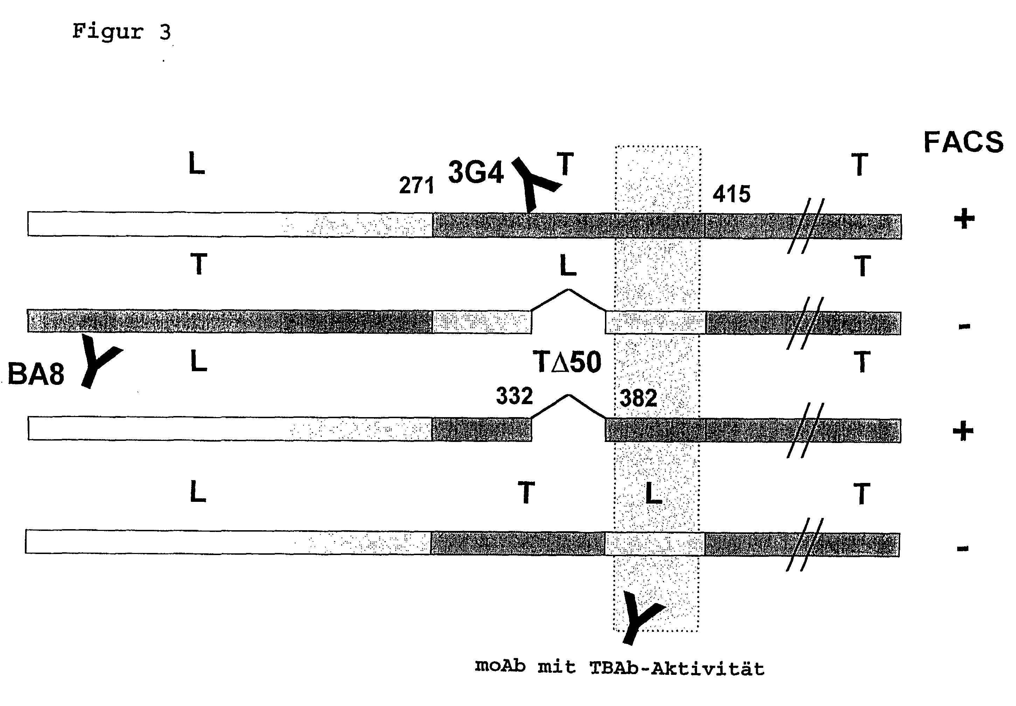

Zur Lokalisierung des Epitops auf der ECD (extrazellulären Domäne) eines von COS-Zellen exprimierten hTSH-R, das von moAbs wie dem moAb 23.1 erkannt wird, wurden Chimären mit Teilsequenzen des LH-Rezeptors (LH-R) und des hTSH-R erzeugt. Und zwar wurden Teilsequenzen der ECD des hTSH-R durch Teilsequenzen des LH-R ersetzt, während die Transmembran-Serpentine des TSH-R jeweils beibehalten wurde. Figur 3 zeigt verschiedene der verwendeten Chimären, wobei mit "L" bezeichnete Sequenzabschnitte aus der ECD des LH-R stammen und daher TRAbs bzw. Anti-TSH-R-moAbs nicht binden sollten, während mit "T" bezeichnete Bereiche aus der ECD des TSH-R stammen und daher potentiell von den untersuchten moAbs erkennbare Teilsequenzen/Strukturen aufweisen können. In der als L-TΔ50-T bezeichneten Chimäre fehlten die nur bei dem hTSH-R vorkommenden 50 Aminosäuren der Positionen 332-382 des hTSH-R. Alle Chimären wurden in den pSVL-Vektor inseriert und in COS-Zellen exprimiert. Der Expressionsgrad der Chimären in den Zelloberflächen der COS-Zellen wurde unter Verwendung verschiedener Anti-TSH-R-moAbs und FACS Analyse im wesentlichen wie unter 1b. beschrieben bestimmt.

### 2. Kompetition von Seren von Autoimmun-Patienten mit dem selektierten ¹²⁵I-markierten Anti-TSH-R-moAb

Für die Kompetitionsversuche wurden den hTSH-R exprimierende transformierte CHO-Zellen (JP19; vgl. Biochem.Biophys.Res. Commun.171: 1044-1050, 1990) auf weißen CulturPlates™ (Packard Instrument Company, Meriden, Connecticut, USA) mit 96 Vertiefungen ausgesät (50.000 Zellen/Vertiefung) und in 50 µl HamF12-Medium, das 0,1% BSA und 10 µl eines im jeweiligen Kompetitierungsversuch eingesetzten Autoimmunserums eines Patienten enthielt, 1 h bei 37°C inkubiert. Anschließend wurden 50 µl des wie oben unter 1e. beschrieben hergestellten und ¹²⁵I-markierten Anti-TSH-R-moAb 23.1 (100.000 cpm/Vertiefung) zugesetzt. 1 h später wurden die Zellen zweimal mit dem gleichen kalten Puffer gewaschen. Es wurden 100 µl MicroScint-20 (Packard Instrument Company, Meriden, Connecticut) zugesetzt, und die an die JP19-Zellen gebundene Radioaktivität wurde in einem TopCount Microplate Scintillation Counter (Packard Instrument Company, Meriden, Connecticut) bestimmt.

### 3. Ergebnisse

Aufgrund der unter 1b.-1d. beschriebenen Testung der Mäuseseren wurde eine gemäß 1a. immunisierte Maus ausgewählt, die klare Zeichen für eine Autoimmun-Hyperthyreose zeigte: T₄ (µg/dl) 11.5 (Kontrollseren zeigten Werte im Bereich von 3.8 bis 4 . 5 ) ; TSAb (pmol/100µL) 8.5 (Kontrollseren <4); TBAb (pmol/100µL) 10.5 (Kontrollseren >22). Splenocyten dieser Maus wurden zur moAb-Produktion gemäß 1e. verwendet. Nach FACS Analyse gemäß 1b. unter Verwendung der Hybridoma-Überstände wurden 12 Anti-TSH-R-moAbs selektiert und getestet.

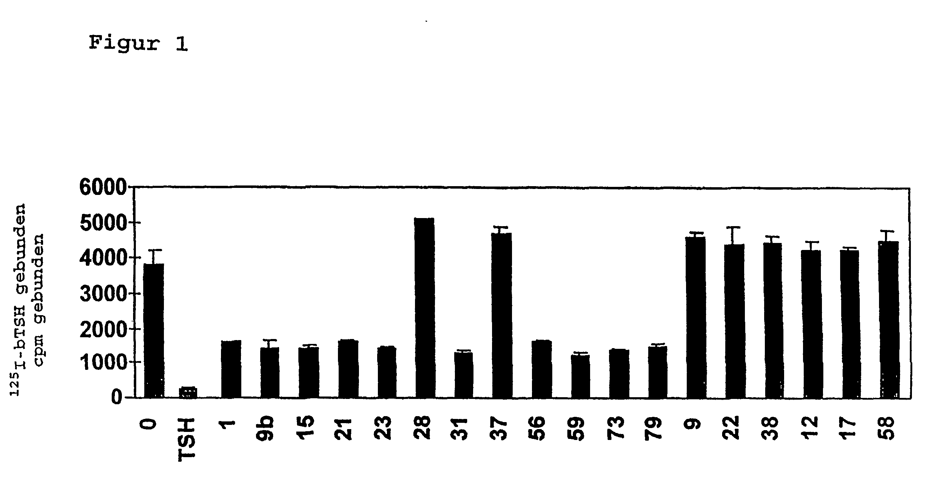

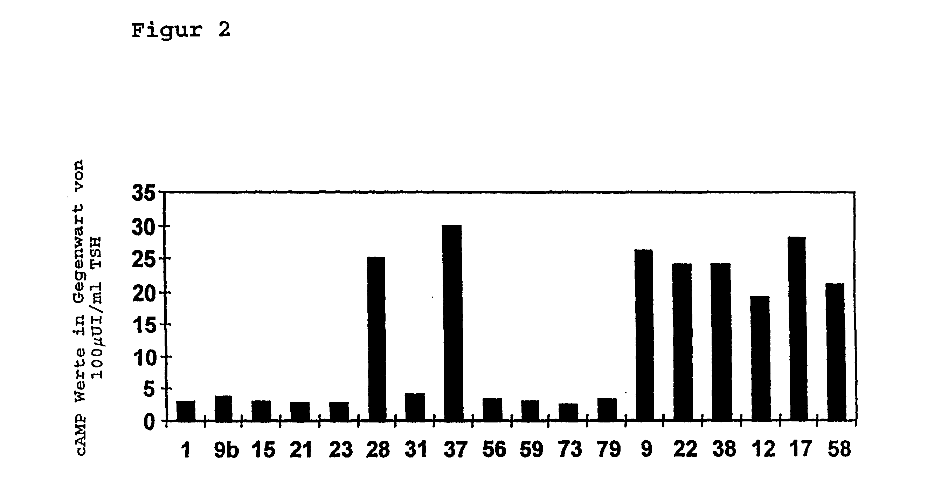

Von den 12 Anti-TSH-R-moAb wiesen 10 mit den Zifferbezeichnungen 1, 9b, 15, 21, 23, 31, 56, 59, 73, 79 im o.g. TBII-Test gemäß 1c. die gesuchte TBII-Aktivität auf (Figur 1) und zeigten im Bio- assay gemäß 1d. eine ausgeprägte TBAb-Wirkung (Figur 2) und keinerlei TSAb-Aktivität. Die TBII-Eigenschaft der genannten 10 moAbs wurde durch Messungen mit dem kommerziellen TRAK Assay® der Fa. B.R.A.H.M.S Diagnostica bestätigt. Die restlichen beiden moAbs (28 und 37) zeigten keine TBII-Aktivität. Kulturüberstände von anderen Hybridomas 9, 22, 38, 12, 17, 58, die mit CHO-Zellen reagierten, jedoch keine Spezifität für den hTSH-R aufwiesen, dienten als Kontrollen.

Die 12 moAbs (Hybridom-Überstände) wurden ergänzend in SDS-PAGE und Western-Blotting-Untersuchungen geprüft, wozu konfluente Zellen GT14, die die extrazelluläre Domäne des TSH-R mit einem Glycosylphosphatidylinosit (GPI)-Anker exprimieren, verwendet wurden. Die 10 moAbs mit TBII-Aktivität erkannten lineare Epitope auf dem reifen, auf der GT14-Zelloberfläche präsentierten hTSH-R, wobei im Falle von 4 dieser moAbs (1, 9b, 31 und 56) die Deglykosylierung des TSH-R mit N-Deglycosidase F das Bindungsverhalten nicht beeinträchtigte.

Unter Verwendung der Chimären gemäß 1f. ergab sich, daß die TLT- und LTLT-Chimären (vgl. Figur 3) von den neuen moAbs mit TBII-Aktivität nicht erkannt wurden. Daraus kann geschlossen werden, daß das von den moAbs erkannte Epitop ein Segment zwischen den Aminosäuren 382 und 415 des hTSH-R, d.h. am extremen C-Terminus der ECD des hTSH-R, umfaßt.

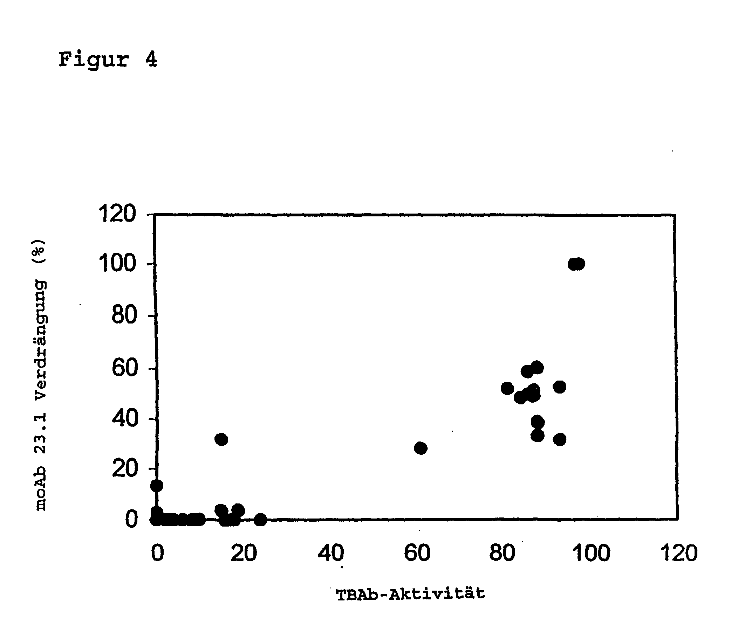

Die gemäß 2. durchgeführten Kompetitionsversuche des 23.1 moAb mit Autoantikörpern aus Seren von 40 Patienten (Nr. 1-40) mit unterschiedlichen TBII/TSAb- und TBAb-Aktivitäten ergaben, daß eine positive Korrelation zwischen der Fähigkeit, die Bindung des ¹²⁵I-moAb 23.1 an die JP19-Zellen zu kompetieren, und der TBAb-Aktivität bestand. Seren mit sehr starker TSAb-Aktivität im Bioassay kompetierten dagegen nicht mit dem ¹²⁵I-moAb 23.1, und es ergab sich auch keine Korrelation der Kompetition mit den gemessenen TBII-Werten. Die Meßergebnisse sind in der nachfolgenden Tabelle 1 sowie in Figur 4 gezeigt.

In Tabelle 1 steht:
(a) % 23.1 Verdrängung für den Wert
   (1 -[¹²⁵I-moAb 23.1 + Serum des jeweiligen Patient (cpm gebunden)] / [¹²⁵I-moAb 23.1 + Normalserum (cpm gebunden)]) x 100
(b) TSAb Aktivität (%) für den Wert
   ([cAMP gemessen mit dem Patientenserum - cAMP gemessen mit Normalserum] / cAMP gemessen mit Normalserum) x 100
(c) TBAb Aktivität (%) für den Wert
   (1 - [cAMP gemessen mit dem Patientenserum plus 200µUI/ml bTSH] / [cAMP gemessen mit Normalserum plus 200µUI/ml bTSH]) x 100

Die der Tabelle 1 und Figur 4 ablesbaren Ergebnisse zeigen, daß nach dem beschriebenen Immunisierungs- und Selektionsverfahren moAb erhalten wurden, die nicht mit TSAb aus Patientenseren kompetieren und es daher gestatten, selektiv den Gehalt von TRAb vom Nicht-TSAb-Typ in einem Patientenserum zu bestimmen, wobei der bestimmte Gehalt der genannten TRAb-Untergruppe positiv mit der TBAb-Aktivität korreliert (vgl. Figur 4) und damit eine Bestimmung von TBAb in Patientenseren mit einem kompetitiven Assay ermöglicht.

**Tabelle 1**

| Patienten-serum Nr. | %23,1 Verdrängung (a) | fT4 pmol/l | TSH | TBII | TSAb (b) | TBAb(c) |
|---|---|---|---|---|---|---|
| | | | [mU/l] | [U/l] | [%] | [%] |
| 1 | 100 | | | >405 | 39% | 98% |
| 2 | 58 | | | >405 | 87% | 86% |
| 3 | 60 | | | >405 | 308% | 88% |
| 4 | 38 | | | >405 | 148% | 88% |
| 5 | 48 | | | >405 | 175% | 84% |
| 6 | 51 | | | >405 | 74% | 87% |
| 7 | 32 | | | >405 | 897% | 15% |
| 8 | 28 | | | >405 | 1459% | 61% |
| 9 | 0 | 16,70 | 0,830 | 95,40 | 357% | 4% |
| 10 | 0 | 27,10 | 0,002 | 56,30 | 333% | 18% |
| 11 | 0 | 4,70 | 20,710 | 56,50 | 292% | 24% |
| 12 | 0 | | | 49,70 | 1733% | 0% |
| 13 | 0 | | | 38,40 | 4182% | 0% |
| 14 | 0 | | | 40,70 | 1857% | 17% |
| 15 | 0 | | | 42,10 | 8000% | 10% |
| 16 | 0 | | | 46,80 | 10000% | 0% |
| 17 | 0 | | | 33.70 | 1333% | 6% |
| 18 | 0 | | | 18,70 | 1929% | 2% |
| 19 | 0 | | | 12,60 | 225% | 3% |
| 20 | 0 | 33,10 | 0,002 | 14,60 | 400% | 24% |
| 21 | 0 | 38,20 | 0,002 | 13,40 | 477% | 8% |
| 22 | 0 | 24,30 | 0,002 | 13,50 | 450% | 16% |
| 23 | 0 | 15,80 | 0,590 | 10,60 | 278% | 9% |
| 24 | 0 | 9,80 | 0.002 | 0,47 | 520% | 0% |
| 25 | 0 | 19,90 | 0,080 | 0,00 | 210% | 0% |
| 26 | 4 | 12,60 | 0,160 | 0,00 | 786% | 15% |
| 27 | 3 | | | 4,30 | 3333% | 19% |
| 28 | 48 | 23,00 | 0,330 | > 405.0 | 180% | 87% |
| 29 | 100 | | | > 405,0 | 50% | 98% |
| 30 | 50 | 22,70 | 0,002 | >405.0 | 110% | 86% |
| 31 | 32 | 11,80 | 7.230 | 19.00 | 160% | 93% |
| 32 | 3 | 7,30 | 21,770 | 156,10 | 160% | 0% |
| 33 | 13 | 8.40 | 0,013 | >405 | 1400% | 0% |
| 34 | 0 | 13,90 | 0,000 | 166,30 | 220% | 10% |
| 35 | 0 | 17,40 | 0,000 | 210,00 | 410% | 0% |
| 36 | 52 | 17,90 | 4,780 | >405 | 160% | 93% |
| 37 | 52 | 10,60 | 54,260 | 320,00 | 110% | 81% |
| 38 | 100 | | | >405 | 50% | 97% |
| 39 | 0 | 14,50 | 8,320 | 126,00 | 110% | 0% |
| 40 | 33 | 19,70 | 2,880 | >405 | 160% | 88% |

## Patentansprüche

1. Verfahren zur Bestimmung von gegen den TSH-Rezeptor (TSH-R) gebildeten Autoantikörpern (TRAb) in einer von einem Patienten gewonnenen biologischen Probe, bei dem man in einer Reaktionsmischung die Probe oder wenigstens eine Antikörperfraktion der Probe in Gegenwart eines ersten Immunreagens in Form eines selektiven Kompetitors mit einem zweiten Immunreagens in Form eines TSH-R-Präparats reagieren läßt, und wobei wenigstens einer von dem selektiven Kompetitor oder dem TSH-R-Präparat markiert oder markierbar ist, und daß man anhand der Bindung zwischen dem TSH-R-Präparat und dem selektiven Kompetitor die Anwesenheit und/oder Menge der gesuchten TRAb in der Probe feststellt, **dadurch gekennzeichnet, daß** man selektiv die Schilddrüse blockierende Autoantikörper (TBAb) dadurch bestimmt, daß der selektive Kompetitor so ausgewählt ist, daß er an solche Bindungsstellen des TSH-R-Präparats bindet, um die er mit den zu bestimmenden TBAb konkurriert.

2. Verfahren nach Anspruch 1, **dadurch gekennzeichnet, daß** der selektive Kompetitor ein anti-TSH-R-Antikörper oder ein Fragment davon ist.

3. Verfahren nach Anspruch 2, **dadurch gekennzeichnet, daß** der anti-TSH-R-Antikörper ein monoklonaler Antikörper ist.

4. Verfahren nach einem der Ansprüche 1 bis 3, **dadurch gekennzeichnet, daß** der selektive Kompetitor ein blockierender anti-TSH-R-Antikörper ist, der im Anschluß an eine genetische Immunisierung einer Maus mit einem hTSH-R-cDNA-Konstrukt hergestellt wurde.

5. Verfahren nach einem der Ansprüche 1 bis 4, **dadurch gekennzeichnet, daß** eines von dem ersten oder zweiten Immunreagens, das ausgewählt ist aus dem TSH-R-Präparat oder dem selektiven Kompetitor, unmarkiert und in einer an eine Festphase immobilisierten oder immobilisierbaren Form eingesetzt wird.

6. Verfahren nach einem der Ansprüche 1 bis 5, **dadurch gekennzeichnet, daß** das TSH-R-Präparat ein rekombinant hergestelltes TSH-R-Präparat ist, das ausgewählt ist aus (i) einem rekombinanten, im wesentlichen vollständigen humanen TSH-R (hTSH-R), (ii) einem TSH-R-Fusionsprotein, das wenigstens die extrazelluläre Domäne des hTSH-R sowie gegebenenfalls Sequenzen für die Markierung und/oder Immobilisierung enthält, und (iii) einer TSH-R-Chimäre, bei der solche Teilsequenzen der extrazellulären Domäne des TSH-R, die für die Bindung von stimulierenden TRAb (TSAb) und/oder neutralen TRAb, benötigt werden, durch inaktive Teilsequenzen ersetzt sind.

7. Verfahren nach Anspruch 4, **dadurch gekennzeichnet, daß** der anti-TSH-R-Antikörper so ausgewählt ist, daß er an ein Epitop im Bereich der Aminosäuren 382-415 der Aminosäurequenz des hTSH-R bindet.

8. Verfahren nach Anspruch 5, **dadurch gekennzeichnet, daß** das immobilisierte Immunreagens ein rekombinantes hTSH-R-Präparat ist, das mit Hilfe eines konformativen Antikörpers, der die Bindung der zu bestimmenden TBAb und des selektiven Kompetitors an das hTSH-R-Präparat nicht behindert, an eine Festphase immobilisiert ist.

9. Verfahren nach einem der Ansprüche 1 bis 8, **dadurch gekennzeichnet, daß** das markierte Immunreagens der selektive Kompetitor ist, der mit Hilfe eines Radioisotops, eines Chemilumineszenz-Labels oder eines Enzyms markiert ist.

10. Verfahren nach einem der Ansprüche 1 bis 8, **dadurch gekennzeichnet, daß** der selektive Kompetitor an eine Festphase immobilisiert ist und daß das als zweites Immunreagens in solubilisierter Form eingesetzte TSH-R-Präparat markiert ist.

11. Verfahren nach einem der Ansprüche 1 bis 4, 6 und 7, **dadurch gekennzeichnet, daß** sowohl das erste als auch das zweite Immunreagens in der Reaktionsmischung dispergiert vorliegen und daß an das den selektiven Kompetitor bildende erste Immunreagenzien eine erste Markierungskomponente gebunden ist, die Teil eines auf Fluoreszenz- oder Chemilumineszenz-Löschung oder -Verstärkung beruhenden Markierungssystems ist, und daß die zweite Markierungskomponente dieses Markierungssystems an das zweite Immunreagens oder einen selektiv an dieses zweite Immunreagens bindenden Antikörper, der die Bindung der zu bestimmenden TBAb und des selektiven Kompetitors an das hTSH-R-Präparat nicht behindert, gebunden ist.

12. Verfahren nach Anspruch 11, **dadurch gekennzeichnet, daß** das Markierungssystem Seltenerdkryptate oder -chelate in Kombination mit einem Fluoreszenz- oder Chemilumineszenzfarbstoff, insbesondere vom Cyanintyp, umfaßt.

## Claims

1. Method for the determination of autoantibodies (TRAb) formed against the TSH receptor (TSH-R) in a biological sample obtained from a patient, in which the sample or at least an antibody fraction of the sample is allowed to react in a reaction mixture in the presence of a first immunoreagent in the form of a selective competitor with a second immunoreagent in the form of a TSH-R preparation, at least one of the selective competitor or the TSH-R preparation being labeled or being capable of being labeled, and the presence and/or amount of the sought TRAb in the sample being determined on the basis of the binding between the TSH-R preparation and the selective competitor, **characterized in that** thyroid-blocking autoantibodies (TBAb) are selectively determined by choosing the selective competitor in such a way that it binds to those binding sites of the TSH-R preparation for which it competes with the TBAb to be determined.

2. Method according to Claim 1, **characterized in that** the selective competitor is an anti-TSH-R antibody or a fragment thereof.

3. Method according to Claim 2, **characterized in that** the anti-TSH-R antibody is a monoclonal antibody.

4. Method according to any of Claims 1 to 3, **characterized in that** the selective competitor is a blocking anti-TSH-R antibody produced subsequent to a genetic immunization of a mouse with an hTSH-R-cDNA construct.

5. Method according to any of Claims 1 to 4, **characterized in that** one of the first or second immunoreagent, which is chosen from the TSH-R preparation or the selective competitor, is unlabeled and is used in a form which is immobilized or can be immobilized on a solid phase.

6. Method according to any of Claims 1 to 5, **characterized in that** the TSH-R preparation is a TSH-R preparation which is prepared by a recombinant method and is selected from (i) a recombinant, substantially complete human TSH-R (hTSH-R), (ii) a TSH-R fusion protein which contains at least the extracellular domains of the hTSH-R and optionally sequences for the labeling and/or immobilization, and (iii) a TSH-R chimera in which those partial sequences of the extracellular domains of the TSH-R which are required for the binding of stimulating TRAb (TSAb) and/or neutral TRAb have been replaced by inactive partial sequences.

7. Method according to Claim 4, **characterized in that** the anti-TSH-R antibody is selected so that it binds to an epitope in the region of the amino acids 382-415 of the amino acid sequence of the hTSH-R.

8. Method according to Claim 5, **characterized in that** the immobilized immunoreagent is a recombinant hTSH-R preparation which is immobilized on a solid phase with the aid of a conformative antibody which does not hinder the binding of the TBAb to be determined and of the selective competitor to the hTSH-R preparation.

9. Method according to any of Claims 1 to 8, **characterized in that** the labeled immunoreagent is the selective competitor, which is labeled with the aid of a radioisotope, of a chemiluminescent label or of an enzyme.

10. Method according to any of Claims 1 to 8, **characterized in that** the selective competitor is immobilized on a solid phase and that the TSH-R preparation used as the second immunoreagent in solubilized form is labeled.

11. Method according to any of Claims 1 to 4, 6 and 7, **characterized in that** both the first and the second immunoreagent are present in dispersed form in the reaction mixture and that a first labeling component which is part of a labeling system based on fluorescence or chemiluminescence extinction or amplification is bound to the first immunoreagent forming the selective competitor, and that the second labeling component of this labeling system is bound to the second immunoreagent or an antibody which binds selectively to this second immunoreagent and does not hinder the binding of the TBAb to be determined and of the selective competitor to the hTSH-R preparation.

12. Method according to Claim 11, **characterized in that** the labeling system comprises rare earth cryptates or chelates in combination with a fluorescent or chemiluminescent dye, in particular of the cyanine type.

## Revendications

1. Procédé de détermination d'auto-anticorps (TRAb) formés contre les récepteurs de la TSH (TSH-R) dans un échantillon biologique recueilli chez un patient, dans lequel on fait réagir, dans un mélange de réaction, l'échantillon ou au moins une fraction d'anticorps de l'échantillon, en présence d'un premier réactif immunologique qui présente la forme d'un compétiteur sélectif, avec un deuxième réactif immunologique qui présente la forme d'une préparation de TSH-R, et dans lequel le compétiteur sélectif et/ou la préparation de TSH-R sont marqués ou peuvent l'être, et en ce qu'à l'aide de la liaison entre la préparation de TSH-R et le compétiteur sélectif, on constate la présence et/ou la quantité du TRAb dans l'échantillon, **caractérisé en ce que** l'on détermine sélectivement l'auto-anticorps (TBAb) qui bloque la thyroïde en sélectionnant le compétiteur sélectif qui se lie à des sites de liaison de la préparation de TSH-R pour entrer en concurrence avec le TBAb à déterminer.

2. Procédé selon la revendication 1, **caractérisé en ce que** le compétiteur sélectif est un anticorps anti-TSH-R ou un fragment de ce dernier.

3. Procédé selon la revendication 2, **caractérisé en ce que** l'anticorps anti-TSH-R est un anticorps monoclonal.

4. Procédé selon l'une des revendications 1 à 3, **caractérisé en ce que** le compétiteur sélectif est un anticorps anti-TSH-R bloquant, qui a été préparé après immunisation génétique d'une souris par un construct d'ADNc de pTSH-R.

5. Procédé selon l'une des revendications 1 à 4, **caractérisé en ce que** l'un parmi le premier et le deuxième réactif immunologique qui est sélectionné à partir de la préparation de TSH-R ou du compétiteur sélectif est non marqué, et est utilisé sous une forme immobilisée ou immobilisable sur une phase solide.

6. Procédé selon l'une des revendications 1 à 5, **caractérisé en ce que** la préparation de TSH-R est une préparation de TSH-R préparée par recombinaison, qui est sélectionnée à partir (i) d'une TSH-R recombinée essentiellement complètement humaine (hTSH-R), (ii) d'une protéine de fusion de TSH-R qui contient au moins le domaine extracellulaire de la hTSH-R, ainsi qu'éventuellement des séquences de marquage et/ou d'immobilisation, et (iii) d'une chimère de TSH-R dans laquelle les séquences partielles des domaines extracellulaires de la TSH-R qui sont nécessaires pour la liaison avec un TRAb stimulant (TSAb) et/ou un TRAb neutre, sont remplacées par des parties inactives de séquences.

7. Procédé selon la revendication 4, **caractérisé en ce que** l'anticorps anti-TSH-R est sélectionné de telle sorte qu'il se lie à un épitope situé dans la zone des acides aminés 382-415 de la séquence d'acides aminés de la hTSH-R.

8. Procédé selon la revendication 5, **caractérisé en ce que** le réactif immunologique immobilisé est une préparation recombinée de pTSH-R qui est immobilisée sur une phase solide à l'aide d'un anticorps conformatif qui n'empêche pas la liaison du TBAb à déterminer et du compétiteur sélectif à la préparation de hTSH-R.

9. Procédé selon l'une des revendications 1 à 8, **caractérisé en ce que** le réactif immunologique marqué est le compositeur sélectif qui est marqué à l'aide d'un radio-isotope; d'un marqueur de chimiluminescence ou d'une enzyme.

10. Procédé selon l'une des revendications 1 à 8, **caractérisé en ce que** le compétiteur sélectif est immobilisé sur une phase solide, et **en ce que** la préparation de TSH-R utilisée comme deuxième réactif immunologique sous forme solubilisée est marquée.

11. Procédé selon l'une des revendications 1 à 4, 6 et 7, **caractérisé en ce que** tant le premier que le deuxième réactif immunologique sont présents sous forme dispersée dans le mélange de réaction, et **en ce qu'**au premier réactif immunologique qui forme le compétiteur sélectif, est lié un composant de marquage qui fait partie d'un système de marquage basé sur l'extinction ou le renforcement de la fluorescence ou de la chimiluminescence, et **en ce que** le deuxième composant de marquage de ce système de marquage est lié au deuxième réactif immunologique ou à un anticorps qui se lie sélectivement à ce deuxième réactif immunologique et qui n'empêche pas la liaison du TBAb à déterminer et du compétiteur sélectif à la préparation de hTSH-R.

12. Procédé selon la revendication 11, **caractérisé en ce que** le système de marquage comporte des cryptats ou chélates de terre rare en combinaison avec un colorant de fluorescence ou de chimioluminescence, en particulier du type cyanine.
